(19) Europäisches Patentamt / European Patent Office / Office européen des brevets

(11) EP 3 260 036 A1

(12) **EUROPEAN PATENT APPLICATION**
published in accordance with Art. 153(4) EPC

(43) Date of publication:
27.12.2017 Bulletin 2017/52

(51) Int Cl.:
*A61B 1/06* (2006.01)

(21) Application number: 16727276.4

(86) International application number:
PCT/JP2016/053621

(22) Date of filing: 08.02.2016

(87) International publication number:
WO 2016/132940 (25.08.2016 Gazette 2016/34)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: 20.02.2015 JP 2015031970

(71) Applicant: HOYA Corporation
Tokyo 160-8347 (JP)

(72) Inventors:
• HAYASHI, Yoshihiro
Tokyo 160-8347 (JP)
• IKEDA, Yuuki
Tokyo 160-8347 (JP)
• MASUKAWA, Yuya
Tokyo 160-8347 (JP)

(74) Representative: Schaumburg und Partner
Patentanwälte mbB
Postfach 86 07 48
81634 München (DE)

(54) **LIGHT SOURCE DEVICE**

(57) A light source device is configured to have a light source configured to emit white light, a rotatable turret provided with multiple kinds of predetermined light passing areas respectively extract light components having different wavelength ranges from the white light incident from the light source, the multiple kinds of predetermined light passing areas being arranged in a circumferential direction of the rotatable turret, and white light passing areas each allowing the white light to pass therethrough being arranged in the circumferential direction of the rotatable turret and between the predetermined wavelength light passing areas, and a driving means configured rotate the rotatable turret so that the multiple kinds of predetermined wavelength light passing areas and the white light passing areas are alternately inserted into a light path of the white light one by one at a timing which is synchronized with a predetermined photographing period.

FIG. 3

EP 3 260 036 A1

## Description

## Technical Field

The present invention relates to a light source device configured emit light toward an object.

## Background Art

There has been known an endoscope system configured to capture particular images. For example, in a WIPO 2014/192781 pamphlet (hereinafter, referred to patent document 1), a concrete configuration of such an endoscope system is disclosed.

The endoscope system described in the patent document 1 has a light source device implemented with a rotatable filter. On the rotatable filter, three optical band-pass filters (i.e., two optical band-pass filters allowing 550nm band light to pass therethrough, and one optical band-pass filter allowing 650nm band light to pass therethrough), and a normal observation filter allowing white light to pass therethrough are arranged in a circumferential direction. A controller causes the rotatable filter at a constant rotation period to sequentially insert the respective filters in a light path of the white light, and sequentially photographs biographic tissues with use of illuminated light which has passed respective filters. The controller generates an image showing distribution of biomolecule of biographic tissue (e.g., an image showing a distribution of oxygen saturation of hemoglobin), and displays the thus generated image showing the distribution within a display screen, together with a normal observation image in an aligned manner.

## Summary of the Invention

In the patent document 1, the filters arranged on the rotatable filter (i.e., four filters: three optical band-pass filters and one normal observation filter) are sequentially inserted in the light path of the white light at a timing synchronous with a frame rate. According to this configuration, however, since photographing of the normal observation image using the normal observation filter is executed at every four frames, a problem has been pointed out such that a refresh rate of the normal observation image is too low.

The present invention is made in view of the above situation, and an object of the present invention is to provided a light source device suitable to suppress lowering of a refresh rate of an image of an object which is illuminated by white light when illuminating light respectively passed through multiple optical filters having different spectral characteristics and white are sequentially used to illuminate the object.

A light source device according to an embodiment of the present invention is provided with a light source configured to emit white light, a rotatable turret on which multiple types of particular wavelength light passing areas to extract light of different wavelength ranges, respectively, which are arranged in a circumferential direction, and white light passing areas allowing the white light to pass therethrough and arranged between the particular wavelength light passing areas are arranged, and a driving means which alternately insert the particular light passing areas and white light passing areas at a timing synchronously with a predetermined photographing period.

According to the one embodiment of the present invention, the white light passing areas are arranged between the particular wavelength light passing areas and the particular wavelength light passing area and the white light passing area are inserted in a light path of the white light alternately, regardless of kinds and arranged numbers of the particular wavelength light passing areas, the white light passed through the white light passing areas is incident on the object at every second photographing period. Accordingly, while multiple kinds of the illuminating light having different spectra are sequentially incident on the object, the white light is incident on the object at every second photographing period. Therefore, it is possible to photograph images of the object illuminated by the white light at every second photographing period with photographing images of the object illuminated by illuminating light of respective spectra.

Further, according to one embodiment of the present invention, the multiple kinds of predetermined wavelength light passing areas may have a first predetermined wavelength light passing area and a second predetermined wavelength light passing area respectively having different light-transmissible wavelength ranges. In such a case, the rotatable turret may be configured such that the first predetermined light passing area, the white light passing area, the second predetermined wavelength light passing area and the white light passing area are arranged in order in the circumferential direction of the rotatable turret.

Further, according to one embodiment of the present invention, the light passing areas may be arranged in the circumferential direction at equal angular pitch.

Further, according to one embodiment of the present invention, each of the white light passing areas may be configured by an optical filter allowing the white light to pass therethrough or an opening provided without an optical filter.

According to one embodiment of the present invention, there is provided a light source device which is suitable to suppress of a refresh rate of the image of the object which is illuminated by white light when illuminating light respectively passed through multiple optical filters having different spectral characteristics and white are sequentially used to illuminate the object.

## Brief Description of the Drawings

Fig. 1 shows enlarged absorption spectrum of hemoglobin at about 550 nm.

Fig. 2 is a block diagram showing a configuration of an electronic endoscope system according to an embodiment of the present invention.

Fig. 3 is a front view, viewed from a converging lens side, of a rotatable filter provided to a processor according to the embodiment of the present invention.

Fig. 4 is a chart showing timings and periods of respective processes regarding a normal observation image and an oxygen saturation distribution image.

**Embodiment for Carrying Out the Invention**

Hereinafter, referring to the drawings, an embodiment of the present invention will be described. It is noted that, in the following description, an electronic endoscope system will be taken as one embodiment of the present invention and described. The electronic endoscope system according to the embodiment is a system capable of qualitatively analyzing biological information (e.g., oxygen saturation) based on multiple images photographed using light of different wavelength ranges and imaging the same.

[Spectral Characteristics of Hemoglobin and Calculation Principle of Oxygen Saturation]

Prior to describing the configuration of the electronic endoscopy system according to the embodiment in detail, spectral characteristics of hemoglobin and a calculation principle the oxygen saturation according to the embodiment will be described.

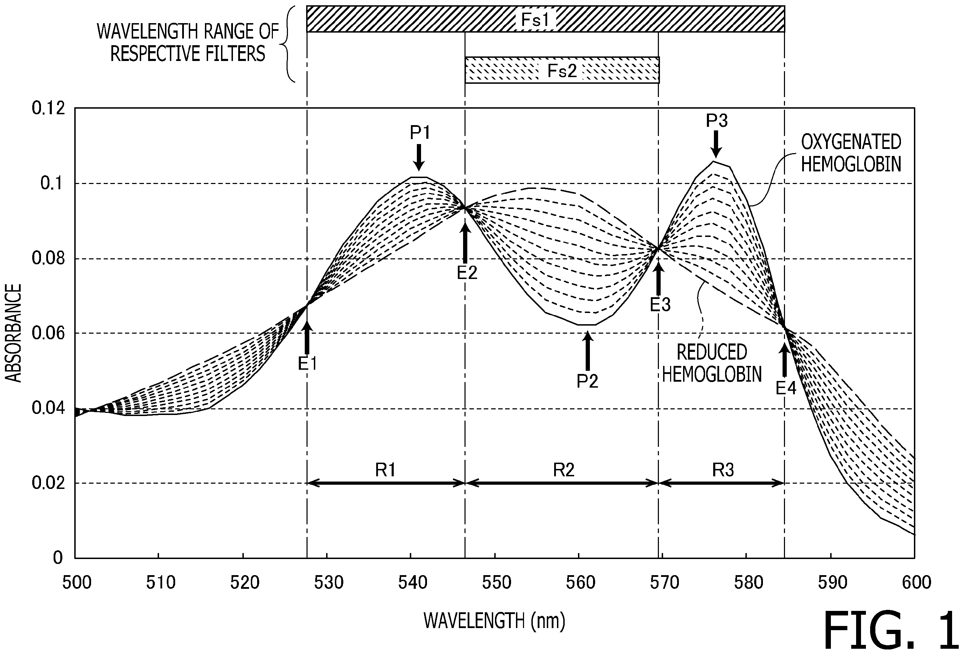

Fig. 1 shows absorption spectrum of the hemoglobin at around 550 nm (nanometer). The hemoglobin has an intense absorption band known as the Q band resulting from porphyrin at 550 nm. The absorption spectrum of the hemoglobin varies depending on the oxygen saturation (i.e., a rate of oxygenated hemoglobin to the entire hemoglobin). A waveform indicated by a solid line in Fig. 1 shows the absorption spectrum when the oxygen saturation is 100 % (i.e., the absorption spectrum of oxygenated hemoglobin HbO), while a waveform indicated by long dash line shows the absorption spectrum when the oxygen saturation is 0 % (i.e., the absorption spectrum of reduced hemoglobin Hb). Waveforms indicated by short dash lines indicate the absorption spectra of the hemoglobin (i.e., mixture of oxygenated hemoglobin and reduced hemoglobin) having intermediate oxygen saturations (i.e., 10%, 20%, 30%, .... 90%).

As shown in Fig. 1, the oxygenated hemoglobin and the reduced hemoglobin have different peak wavelengths in the Q band. Specifically, the oxygenated hemoglobin has an absorption peak P1 at around the wavelength of 542 nm, and another absorption peak P3 at around the wavelength of 578 nm. The reduced hemoglobin has an absorption peak P3 at around the wavelength of 558 nm. Since Fig. 1 shows a two-component absorption spectra in which sum of densities of respective components (i.e., the oxygenated hemoglobin and the reduced hemoglobin) is constant, isosbestic points E1, E2, E3 and E4, at each of which the absorbance is constant regardless of densities of respective components (i.e., the oxygenated hemoglobin and the reduced hemoglobin), appear in Fig. 1. In the following description, a wavelength range between isosbestic points E1 and E2 will be referred to as a wavelength range R1, a wavelength range between isosbestic points E2 and E3 will be referred to as a wavelength range R2, and a wavelength range between isosbestic points E3 and E4 will be referred to as a wavelength range R3. Further, a wavelength range between isosbestic points E1 and E4 (i.e., a combination of wavelength ranges R1, R2 and R3) will be referred to as a wavelength range R0.

As shown in Fig. 1, between adjacent isosbestic points, absorbance monotonously increases or decreases with respect to the oxygen saturation. Further, between adjacent isosbestic points, absorption of the hemoglobin changes substantially linearly with respect to the oxygen saturation.

Specifically, the absorptions $A_{R1}$ and $A_{R3}$ of the hemoglobin in the wavelength ranges R1 and R3 monotonously increase with respect to the density of the oxygenated hemoglobin (i.e., the oxygen saturation), while the absorption $A_{R2}$ of the hemoglobin in the wavelength range R2 monotonously increases with respect to the reduced hemoglobin (i.e., 1-oxygen saturation). Therefore, index X defined by formula (1) below linearly and monotonously increases with respect to the density of the oxygenated hemoglobin (i.e., the oxygen saturation).

$$X = (A_{R1} + A_{R3}) - A_{R2} \quad \cdots\cdots \text{(formula 1)}$$

Accordingly, if quantitative relationship between the oxygen saturation and index X is experimentally obtained in advance, the oxygen saturation can be calculated based on the value of index X.

[Configuration of Electronic Endoscope System]

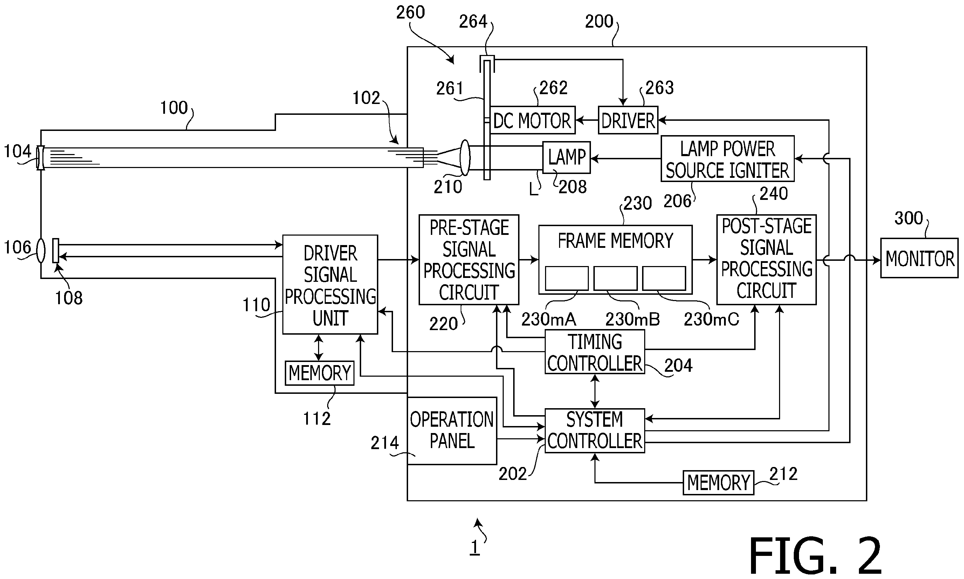

Fig. 2 is a block diagram showing a configuration of the electronic endoscopy system 1 according to the embodiment. As shown in Fig. 2, the electronic endoscope system 1 has an electronic scope 100, a processor 200 and a monitor 300.

The processor 200 has a system controller 202 and a timing controller 204. The system controller 202 is configured to execute various programs stored in a memory 212 to integrally control the electronic endoscope system 1 entirely. The system controller 202 is connected to an operation panel 214. The system controller 202 is configured to change operations of the electronic endoscope system 1 and parameters of respective operations in accordance with instructions by an operator input through the operation panel 214. The timing controller 204 outputs clock pulses which adjust timings of operations in respective components to circuits in the electronic endoscope system 1.

A lamp 208 is configured emit illumination light L after being started with use of a lamp power igniter 206. The lamp 208 is, for example, a high-brightness lame such as a xenon lamp, a halogen lamp, a mercury lamp and a metal halide lamp or an LED (light emitting diode). The illumination light L is light having spectrum ranging from a visible light range to an infrared light range (or, white light including at least the visible light range).

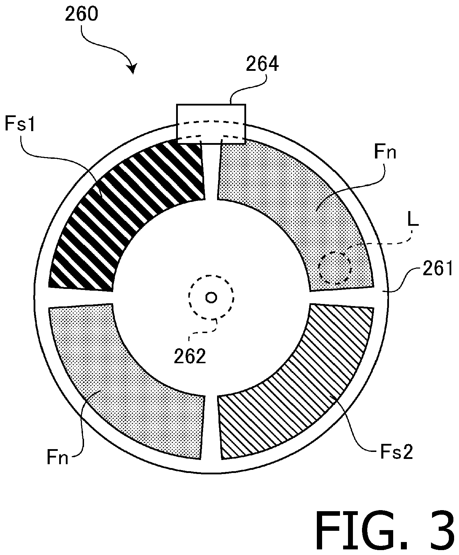

The illumination light L irradiated by the lamp 208 is incident on the rotatable filter part 260. Fig. 3 is a front view of the rotational filter part 260 viewed from a converging lens 210 side. As shown in Fig. 3, the rotational filter part 260 has a rotatable turret 261, a DC (direct current) motor 262, a driver 263 and a photo interrupter 264. The rotatable turret 261 is provided with four optical filters. Specifically, on the rotatable turret 261, a normal light observation filter Fn (for the white light), a first oxygen saturation observing filter Fs1, another normal observation filter Fn and a second oxygen saturation observation filter Fs2 are arranged in a circumferential direction in this order. Each of the optical filters is fan-shaped, and arranged at an angular pitch (e.g., a ninety-degree angular pitch in this example) corresponding to a photographing period (i.e., a frame period). Each optical filter is a dielectric multilayer film filter. However, different types of optical filter (e.g., an etalon filter using a dielectric multilayer film as a reflection film). It is noted that, in the following description, a term "frame" could be replaced with a term "field." According to the embodiment, a frame period and a field period are 1/30 second and 1/60 second, respectively.

The first oxygen saturation observation filter Fs1 is an optical band pass filter which selectively transmits 550nm band light. As shown in Fig. 1, the first oxygen saturation observation filter Fs1 has a spectral characteristic of transmitting light of which wavelength range is from the isosbestic point E1 to the isosbestic point E4 (i.e., the wavelength range of R0) with low loss, while shield light of the other wavelength ranges. The second oxygen saturation observation filter Fs2 has a spectral characteristic of transmitting light of which wavelength range is from the isosbestic point E2 to the isosbestic point E3 (i.e., the wavelength range of R2) with low loss, while shields light of the other wavelength ranges.

The normal observation filter Fn is an ultraviolet cut filter. The normal observation filter Fn may be replaced with a mere opening (with no optical filter) or a slit (with no optical filter) which also has a diaphragm function.

In the following description, for the sake of explanation, the illumination light L transmitted through the first oxygen saturation observation filter Fs1 will be referred to as "first oxygen saturation observation light Ls1" and the illumination light L transmitted through the second oxygen saturation observation filter Fs2 will be referred to as "second oxygen saturation observation light Ls2," and the illumination light L transmitted through the normal observation filter Fn will be referred to as "normal light Ln."

The driver 263 drives the DC motor 262 under control of the system controller 202. The rotatable filter part 260 sequentially inserts each of the optical filters, the normal observation filter Fn, the first oxygen saturation observation filter Fs1, the normal observation filter Fn and the second oxygen saturation observation filter Fs2, as the rotatable turret 261 is rotated by the DC motor 262. Then, from the illumination light L irradiated by the lamp 208, a plurality of illumination light having different spectra is extracted sequentially at timings synchronously with the frame period. Specifically, the rotatable turret 261 extracts, circularly, the first oxygen saturation observation light Ls1 from the first oxygen saturation observation filter Fs1, the normal light Ln from the normal observation filter Fn, the second oxygen saturation observation light Ls2 from the second oxygen saturation observation filter Fs2 and the normal light Ln from the normal observation filter Fn. A rotary position and rotary phase of the rotatable turret 261 are controlled by detecting opening (not shown) formed at outer peripheral portions of the rotatable turret 261 with the photo interrupter 264.

The illumination light (i.e., the first oxygen saturation observation light Ls1, the second oxygen saturation observation light Ls2 and the normal light Ln) extracted from the rotatable filter part 260 is converged, by the converging lens 210 with its light amount being adjusted to an appropriate amount by a blade diaphragm (not shown), on a light incident surface of an LCB (light carrying bundle) 102 and introduced inside the LCB 102.

The illumination light introduced inside the LCB 102 (i.e., the first oxygen saturation observation light Ls1, the second oxygen saturation observation light Ls2 and the normal light Ln) propagates inside the LCB 102, emitted from an emission surface of the LCB 102 arranged at a tip of the electronic endoscope 100, and is incident on the object through a light distribution lens. Accordingly, the object is sequentially illuminated by the first oxygen saturation observation light Ls1, the normal light Ln, the second oxygen saturation observation light Ls2 and the normal light Ln. Reflected light from the object which is illuminated by the illumination light forms an optical image on a light receiving surface of a solid sate imaging device 108.

The solid state imaging device 108 is a single-CCD (charge coupled device) color image sensor having Bayer type pixel arrangement. The solid state imaging device 108 accumulates an optical image formed on each pixel on the light receiving surface as electric charges corresponding to the intensity of light, generates R (red), G (green) and B (blue) image signals and outputs the same. It is noted that the solid state imaging device 108 needs not be limited to the CCD image sensor, and may be replaced with CMOS (complementary metal oxide semiconductor) image sensor or other types of imaging devices. Further, the solid state imaging device 108 may be one mounting complementary color filters.

Timings at which the rotatable filter part 260 switches the illumination light (i.e., the first oxygen saturation observation light Ls1, the second oxygen saturation observation light Ls2 and the normal light Ln) are synchronous with timings at which imaging periods (i.e., the frame periods) by the solid state imaging device 108 are switched. Accordingly, in one frame period, the solid state imaging device 108 receives the first oxygen saturation observation light Ls1, and generates a first oxygen saturation observation image signal Ss1 and outputs the same, and in a subsequent one frame period, the solid state imaging device 108 receives the normal light Ln, and generates a normal observation image signal Sn and outputs the same, then, in the subsequent one frame period, the solid state imaging device 108 receives the second oxygen saturation observation light Ls2, and generates a second oxygen saturation observation image signal Ss2 and outputs the same, and in the subsequent one frame period, the solid state imaging device 108 receives the normal light Ln, and generates the normal observation image signal Sn and outputs the same. That is, the first oxygen saturation observation image signal Ss1, the normal observation image signal Sn, the second oxygen saturation observation image signal Ss2 and the normal observation image signal Sn are sequentially generated and output by the solid state imaging device 108.

Inside a connecting part of the electronic endoscope 100, a driver signal processing circuit 110 is provided. Into the driver signal processing part 110, the image signals Ss1, Sn, Ss2 and Sn are sequentially input at the frame period from the solid state imaging device 108. The driver signal processing circuit 110 applies a predetermined processing to the image signals input from the solid state imaging device 108 and output the same to a pre-stage signal processing circuit 220 of the processor 200.

The driver signal processing circuit 110 is also configured to access the memory 112 and retrieves device-specific data of the electronic endoscope 100. Examples of device-specific information of the electronic endoscope 100 stored in the memory 112 include the number of pixels, sensitivity, operable frame rate and a model number of the solid state imaging device 108. The driver signal processing circuit 110 transmits the device-specific information retrieved from the memory 112 to the system controller 202.

The system controller 202 executes various types of operations based on the device-specific information of the electronic endoscope 100, and generates a control signal. With use of the generated control signal, the system controller 202 controls operations and timings of various circuits inside the processor 200 so that processing suitable to the electronic endoscope connected to the processor 200 is executed.

The timing controller 204 supplies a clock pulse to the driver signal processing circuit 110 in accordance with the timing control of the system controller 202. The driver signal processing circuit 110 drives and controls the solid state imaging device 108 at timings synchronously with the frame rate of the image processed by the processor 200.

The pre-stage signal processing circuit 220 applies a predetermined signal processing to the image signals Ss1, Sn, Ss2 and Sn, which are sequentially received from the driver signal processing circuit 110 at one-frame period, and transmits the same to the frame memory 230.

The frame memory 230 includes three frame memories (i.e., a first oxygen saturation observation memory 230mA, a second oxygen saturation observation memory 230mB and a normal observation memory 230mC). In each of the frame memories, the image signals transmitted from the pre-stage signal processing circuit 220 are written (i.e., overwritten). Specifically, the first oxygen saturation observation image signal Ss1 is written in the first oxygen saturation observation memory 230mA, the second oxygen saturation observation image signal Ss2 is written in the second oxygen saturation observation memory 230mB, and the normal image observation signal Sn is written in the normal observation memory 230mC. The frame memory 230 outputs image signals for respective frame memories to the post-stage signal processing circuit 240 synchronously with the clock pulse received from the timing controller 204.

The post-stage signal processing circuit 240 calculates the index X, with use of formula (1) above, based on the first oxygen saturation observation signal Ss1 and the second oxygen saturation observation signal Ss2 transmitted from the frame memory 230.

In a non-volatile memory (not shown) provided to the pre-stage signal processing circuit 240, a numerical table indicating a quantitative relationship between the oxygen saturation values of the hemoglobin, which have been experimentally obtained in advance, and values of the index X. The post-stage signal processing circuit 240 obtains, referring to the numerical table, the oxygen saturation $SatO_2(x, y)$ corresponding to the value of the index X calculated with use of the formula (1). The post-stage signal processing circuit 240 then generates image data (i.e., oxygen saturation distribution image data), in which values of the oxygen saturation SatO2(x, y) multiplied by a predetermined constant are pixel values of respective pixels(x, y).

The post-stage signal processing circuit 240 also generates normal observation image data with use of the normal observation image signal Sn transmitted from the frame memory 230.

The post-stage signal processing circuit 240 converts the oxygen saturation distribution image data and the normal observation image data into predetermined video format signals. The thus converted video format signals are transmitted to the monitor 300. Then, the oxygen saturation distribution image and the normal observation image are displayed on a display screen of the monitor 300.

The operator can set a display form of the observation image by operating the operation panel 214. As the display form of the observation image, there are a form of displaying the same size of the oxygen saturation distribution image and the normal observation image side by side in one screen, a form of displaying one of the images as a parent image while the other as a child image, a form of displaying only one of the images selected by the operation of the operator as a full screen display, and a form of displaying the oxygen saturation distribution image on the normal observation image in an overlaid manner. Further, on the display screen, information regarding the endoscopic observation which is input through the operation panel 214 (e.g., a name of the operator, date and time of observation, a type of illumination light user for observation, etc.) can be displayed in a superimposed manner.

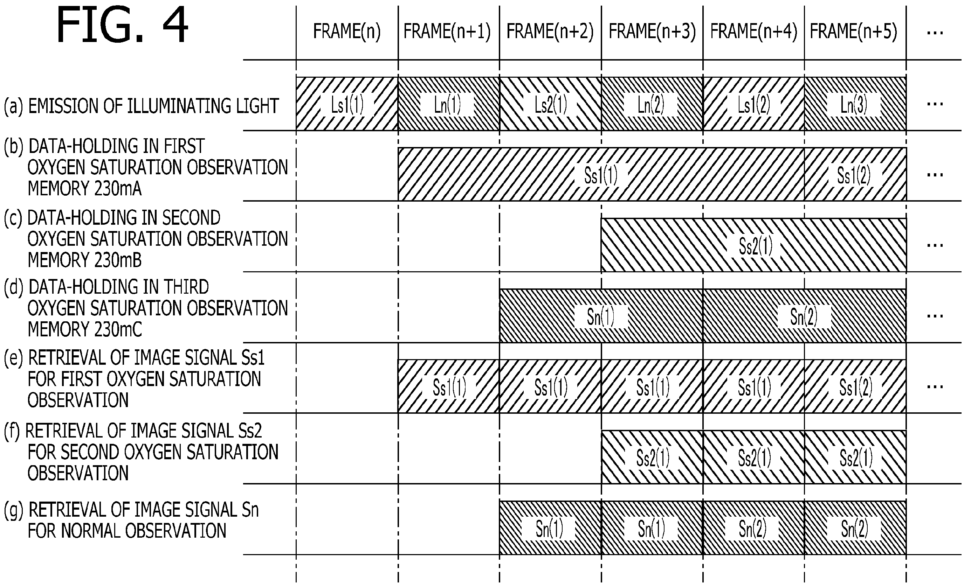

Figs. 4(a) - 4(g) are charts showing processes, timings and periods related to the normal observation image and the oxygen saturation distribution image. Fig. 4(a) shows periods during which the object (inside a body cavity) is illuminated by the illumination light (i.e., the first oxygen saturation observation light Ls1, the second oxygen saturation observation light Ls2 and the normal light Ln). As shown in Fig. 4(a), the first oxygen saturation observation light Ls1 and the second oxygen saturation observation light Ls2 are irradiated toward the object at ever four frames, while the normal light Ln is irradiated toward the object at every two frames. Fig. 4(b) shows writing timings at which the first oxygen observation image signal Ss1 is written in the first oxygen saturation observation memory 230mA, and data-holding periods. Fig. 4(c) shows writing timings at which the second oxygen observation image signal Ss2 is written in the second oxygen saturation observation memory 230mB, and data-holding periods. Fig. 4(d) shows writing timings at which the normal observation image signal Sn is written in the normal observation memory 230mC, and data-holding periods. Fig 4(e), Fig. 4(f) and Fig. 4(g) show periods during which the first oxygen saturation observation image signal Ss1, the second oxygen saturation observation image signal Ss2 and the normal observation image signal Sn are retrieved from the frame memory 230, respectively.

In the example shown in Fig. 4, a writing start timing of the image signal to the frame memory 230 (see Fig. 4(b) - Fig. 4(d)) is indicated to delay by one frame period with respect to the illumination start timing of the illumination light (see Fig. 4(a)), and the retrieval start timing of the image signal (see Fig. 4(e) - Fig. 4(g)) is indicated to be the same timing as the writing start timing of the image signal to the frame memory. It is noted that the above timings are indicated at good places as an aid of convenient explanation. The processes of illuminating, writing and retrieving are not started exactly at the timings shown in Fig. 4(a) - Fig. 4(g), but started sequentially with slight delays. It is noted that, although the execution period of each of the processes of illuminating, holding and retrieving is uniformly indicated as one frame period in the example shown in Fig. 4, these execution periods are indicated to be good periods as an aid of convenient explanation. Practically, the executions periods of the illuminating, holding and retrieving processes are different.

In Fig. 4(a), the illuminating period with the illumination light is indicated by an indication having a format of "a mark of illuminating light (ordinal number)." For example, "Ls1(1)" in Fig. 4(a) represents a first illuminating period with the first oxygen saturation observation light Ls1, while "Ls1(2)" represents a second illuminating period with the first oxygen saturation observation light Ls1.

In each of Fig. (b) - Fig. 4(d), the writing timing and the holding period of the image signal in the frame memory 230 are indicated by an indication having a format of "a mark of the image signal (ordinal number)" for the sake of explanation. For example, in Fig. 4(b), "Ss1(1)" represents a timing of writing the first oxygen saturation observation image signal Ss1 of the object illuminated with the first oxygen saturation observation signal Ls1 (first time) in the first oxygen saturation observation memory 230mA and its holding period, while, "Ss1(2)" represents the timing of writing the first oxygen saturation observation image signal Ss1 of the object illuminated with the first oxygen saturation observation signal Ls1 (second time) in the first oxygen saturation observation memory 230mA and its holding period.

In each of Fig. 4(e) - Fig. 4(g), the retrieval period of the image signal is indicated by an indication having a format of "a mark of the image signal (ordinal number)" for the sake of explanation. For example, in Fig. 4(e), "Ss1(1)" represents a retrieval period in which the first oxygen saturation observation image signal Ss1 (first time) is retrieved from the first oxygen saturation observation memory 230mA, while "Ss1(2)" represents a retrieval period in which the first oxygen saturation observation image signal Ss1 (second time) is retrieved from the first oxygen saturation observation memory 230mA.

According to the embodiment, as shown in Fig. 4, the first oxygen saturation observation image signal Ss1, the second oxygen saturation observation image signal Ss2 are updated at every four frames, and the normal observation image signal Sn is updated at every two frames. Therefore, on the display screen of the monitor 300, the oxygen saturation distribution image obtained with use of two types of optical filters having different spectral characteristics (i.e., the first oxygen saturation observation filter Fs1, and the second oxygen saturation observation filter Fs2) is displayed, while the normal observation image of which refresh rate is suppressed from lowering (i.e., concretely, updating frequency is every two frames) is also displayed. Since the lowering of the refresh rate of the normal observation image is suppressed, for example, stress the operator may feel when viewing the display screen can be reduced.

The foregoing is an exemplary explanation of the present invention. The embodiment of the present invention needs not be limited to the explained one, but various modification can be made within a scope of technical ideas of the present invention. For example, configurations of appropriate combinations of embodiments exemplary indicated or obvious in accordance with the specification should be included in the embodiments of the present invention.

In the above-described embodiment, the light source device is accommodated in the processor 200, in other embodiments, the processor 200 and the light source device may be separate configurations. In such a case, a wired or wireless communication means to transmit/receive timing signals between the processor 200 and the light source device may be provided.

It is also noted that, the light combined with the normal light Ln needs not be limited to light for the oxygen saturation observation (i.e., the first oxygen saturation observation light Ls1 and the second oxygen saturation observation light Ls2). As examples of the light combined with the normal light Ln include, for example, infrared light for an infrared light observation, excitation light for fluorescent observation, narrow band light for narrow band image observation, and the like.

In the above-described embodiment, the rotatable filter part 260 is proved on the lamp 208 side, and filtering is effected with respect to the illumination light L. The invention needs not be limited to such a configuration. For example, a configuration in which the rotatable filter part 260 is provided on the solid state imaging device 108 side and filtering is effected with respect to the light returned from the object may be used.

**Claims**

1. A light source device, comprising:

   a light source configured to emit white light;
   a rotatable turret provided with multiple kinds of predetermined light passing areas respectively extract light components having different wavelength ranges from the white light incident from the light source, the multiple kinds of predetermined light passing areas being arranged in a circumferential direction of the rotatable turret, and white light passing areas each allowing the white light to pass therethrough being arranged in the circumferential direction of the rotatable turret and between the predetermined wavelength light passing areas; and
   a driving means configured rotate the rotatable turret so that the multiple kinds of predetermined wavelength light passing areas and the white light passing areas are alternately inserted into a light path of the white light one by one at a timing which is synchronized with a predetermined photographing period.

2. The light source device according to claim 1, wherein the multiple kinds of predetermined wavelength light passing areas comprises a first predetermined wavelength light passing area and a second predetermined wavelength light passing area respectively having different light-transmissible wavelength ranges;
   wherein the rotatable turret is configured such that the first predetermined light passing area, the white light passing area, the second predetermined wavelength light passing area and the white light passing area are arranged in order in the circumferential direction of the rotatable turret.

3. The light source device according to claim 1 or claim 2, wherein the light passing areas are arranged in the circumferential direction at equal angular pitch.

4. The light source according to any one of claims 1 - 3, wherein each of the white light passing areas is configured by an optical filter allowing the white light to pass therethrough or an opening provided without an optical filter.

FIG. 1

FIG. 2

FIG. 3

FIG. 4

| | FRAME(n) | FRAME(n+1) | FRAME(n+2) | FRAME(n+3) | FRAME(n+4) | FRAME(n+5) | ... |
|---|---|---|---|---|---|---|---|
| (a) EMISSION OF ILLUMINATING LIGHT | Ls1(1) | Ln(1) | Ls2(1) | Ln(2) | Ls1(2) | Ln(3) | ... |
| (b) DATA-HOLDING IN FIRST OXYGEN SATURATION OBSERVATION MEMORY 230mA | | Ss1(1) | | | | Ss1(2) | ... |
| (c) DATA-HOLDING IN SECOND OXYGEN SATURATION OBSERVATION MEMORY 230mB | | | | Ss2(1) | | | ... |
| (d) DATA-HOLDING IN THIRD OXYGEN SATURATION OBSERVATION MEMORY 230mC | | | Sn(1) | | Sn(2) | | ... |
| (e) RETRIEVAL OF IMAGE SIGNAL Ss1 FOR FIRST OXYGEN SATURATION OBSERVATION | | Ss1(1) | Ss1(1) | Ss1(1) | Ss1(1) | Ss1(2) | ... |
| (f) RETRIEVAL OF IMAGE SIGNAL Ss2 FOR SECOND OXYGEN SATURATION OBSERVATION | | | | Ss2(1) | Ss2(1) | Ss2(1) | |
| (g) RETRIEVAL OF IMAGE SIGNAL Sn FOR NORMAL OBSERVATION | | | Sn(1) | Sn(1) | Sn(2) | Sn(2) | |

## INTERNATIONAL SEARCH REPORT

| International application No. |
| --- |
| PCT/JP2016/053621 |

**A. CLASSIFICATION OF SUBJECT MATTER**
*A61B1/06*(2006.01)i

According to International Patent Classification (IPC) or to both national classification and IPC

**B. FIELDS SEARCHED**

Minimum documentation searched (classification system followed by classification symbols)
A61B1/00-1/32

Documentation searched other than minimum documentation to the extent that such documents are included in the fields searched
Jitsuyo Shinan Koho          1922-1996    Jitsuyo Shinan Toroku Koho    1996-2016
Kokai Jitsuyo Shinan Koho    1971-2016    Toroku Jitsuyo Shinan Koho    1994-2016

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2011-188929 A  (Olympus Corp.),<br>29 September 2011 (29.09.2011),<br>paragraphs [0037] to [0050]; fig. 7 to 10<br>(Family: none) | 1-4 |

☐ Further documents are listed in the continuation of Box C.          ☐ See patent family annex.

| | |
| --- | --- |
| * Special categories of cited documents:<br>"A" document defining the general state of the art which is not considered    to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br>21 April 2016 (21.04.16) | Date of mailing of the international search report<br>10 May 2016 (10.05.16) |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)